# DEMANDE DE BREVET EUROPEEN

(11) **EP 1 029 530 A1**
(43) Date de publication de la demande: **23.08.2000**
(21) Numéro de dépôt: 00400269.7
(22) Date de dépôt: 01.02.2000
(51) Int. Cl.: A61K 7/32, A61K 7/48

(54) **Utilisation de dérivés substitués en 3 du stilbene comme actifs déodorants dans les compositions cosmétiques**

(30) Priorité: 17.02.1999 FR 9901931
(71) Demandeur: L'OREAL, 75008 Paris (FR)
(72) Inventeur: Courbiere, Christophe, 92110 Clichy (FR); Pruche, Francis, 75018 Paris (FR); Kravtchenko, Sylvain, 75012 Paris (FR)
(74) Mandataire: Miszputen, Laurent

(57) **Abrégé**

La présente invention a pour objet l'utilisation du 3,5-dihydroxystilbène ou du 3-hydroxystilbène et/ou de leurs dérivés comme actifs déodorants dans la préparation de compositions cosmétiques, les compositions cosmétiques déodorantes contenant ces composés, et l'utilisation desdites compositions pour l'application topique humaine. Elle a aussi pour objet de nouveaux dérivés substitués en 3 du stilbène et les compositions cosmétiques les comprenant.

## Description

La présente invention a pour objet l'utilisation du 3,5-dihydroxystilbène ou du 3-hydroxystilbène et/ou de leurs dérivés comme actifs déodorants dans la préparation d'une composition cosmétique, la composition cosmétique déodorante contenant ces composés, et l'utilisation de ladite composition pour l'application topique humaine.

Elle concerne également un procédé pour traiter les odeurs axillaires humaines consistant à appliquer sur la surface axillaire une quantité efficace de ladite composition.

Dans le domaine cosmétique, il est bien connu d'utiliser en application topique, des produits déodorants contenant des substances actives de type antitranspirant ou de type bactéricide pour diminuer, voire supprimer, les odeurs axillaires généralement désagréables.

Les substances antitranspirantes ont pour effet de limiter le flux sudoral. Elles sont généralement constituées de sels d'aluminium qui, d'une part, sont irritants pour la peau et qui, d'autre part, diminuent le flux sudoral en modifiant la physiologie cutanée, ce qui n'est pas satisfaisant.
Les substances bactéricides inhibent le développement de la flore cutanée responsable des odeurs axillaires. Parmi les produits bactéricides, le plus couramment employé est le Triclosan (2,4,4'-trichloro-2'-hydroxydiphényléther), qui présente l'inconvénient de modifier de façon importante l'écologie de la flore cutanée, d'être inhibé par certains composés, comme par exemple les tensioactifs non-ioniques, couramment utilisés dans la formulation de compositions cosmétiques. Par ailleurs, le caractère insoluble du Triclosan dans l'eau ne permet pas son incorporation dans des formules essentiellement aqueuses.

Dans le but d'obtenir une efficacité à long terme, on recherche de nouveaux produits exerçant une action déodorante, c'est-à-dire des produits qui soient capables de modifier, de réduire et/ou d'ôter ou de prévenir le développement des odeurs corporelles (celle définition est donnée dans l'ouvrage 〈〈 Cosmetic Science and Technology Series 〉〉 - 1988 / Volume 7 chap.10 - IIIc). En outre, on recherche des produits qui ne présentent pas les inconvénients des substances actives utilisées dans l'art antérieur.

On sait que certains composés du stilbène, tels que le 4-hydroxystilbène et le 3,5,4'-stilbène (ou Resvératrol), permettent de limiter le développement de l'odeur de sueur. Toutefois l'efficacité de ces molécules est très nettement insuffisante.

Après de nombreuses recherches menées sur la question, la Demanderesse a maintenant découvert, de façon inattendue et surprenante, que les composés de formule (I), définie ci-après, présentent la propriété de prévenir le développement des odeurs de sueur corporelle, sans les inconvénients des substances actives antérieurement employées dans les compositions déodorantes, et avec l'avantage, pour certains de ces composés, d'être hydrosolubles dans des proportions intéressantes et suffisantes pour être aisément formulables, notamment dans les compositions cosmétiques à base d'eau pour l'application topique humaine.

Cette découverte est à la base de la présente invention.

La présente invention a ainsi pour premier objet, l'utilisation des composés de formule (I) suivante : 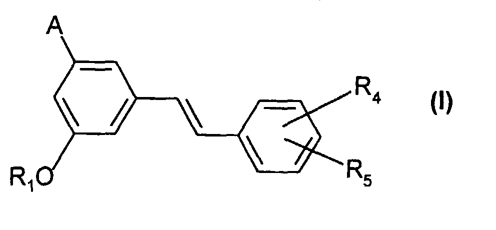 dans laquelle :
**A** désigne un atome d'hydrogène, ou un radical OR₂,
**R**_{**1**} et **R**_{**2**}, identiques ou différents, désignent un atome d'hydrogène ; un radical alkyle en C₁-C₄ ; un radical acyle R₃CO dans lequel R₃ désigne un radical hydrocarboné en C₁-C₃₀, linéaire ou ramifié, saturé ou non saturé, hydroxylé ou non hydroxylé, carboxylé ou non carboxylé ;
un radical PO (OX₁) (OX₂) ou un radical SO₂ (OX₃) dans lesquels, X₁, X₂, X₃, identiques ou différents, désignent un atome d'hydrogène ou un cation monovalent de métal alcalin ou d'NH4⁺ , X₁ et/ou X₂ pouvant également désigner un cation de métal divalent ;
un radical glycosyle ; un radical uronyle ;
**R**_{**4**} et **R**_{**5**} _{**,**} identiques, désignent un atome d'hydrogène ou un radical hydroxyle,
comme actif déodorant, dans une composition cosmétique.

Elle a pour second objet, une composition cosmétique déodorante comprenant au moins un composé de formule (I) décrit ci-avant.

Elle a également pour objet, l'utilisation de ladite composition, dans des, ou pour la fabrication de, produits cosmétiques déodorants destinés à l'application topique humaine.

Elle a aussi pour objet, l'utilisation d'au moins un composé de formule (I) décrite ci-avant, comme agent inhibiteur sélectif de certaines espèces bactériennes constitutives de la flore cutanée humaine, et en particulier Corynebacterium xerosis.

Elle a enfin pour objet, un procédé pour traiter les odeurs axillaires humaines, consistant à appliquer sur la surface axillaire une quantité efficace de ladite composition.

Elle a pour autre objet, de nouveaux composés de formule (I) telle que définie ci-dessus et dans laquelle, A désigne un atome d'hydrogène, ou un radical OR₂, R₁ et R₂, identiques ou différents, désignent un radical acyle R₃CO dans lequel R₃ désigne un radical hydrocarboné en C₄-C₃₀, linéaire ou ramifié, saturé ou non saturé, hydroxylé ou non hydroxylé, carboxylé ou non carboxylé; un radical PO (OX₁) (OX₂) ou un radical SO₂ (OX₃) dans lesquels, X₁, X₂, X₃, identiques ou différents, désignent un atome d'hydrogène ou un cation monovalent de métal alcalin ou d'NH4⁺ , X₁ et/ou X₂ pouvant également désigner un cation de métal divalent; un radical uronyle.

Elle a aussi pour autre objet une composition cosmétique comprenant au moins un composé de formule (I) définie ci-dessus et dans laquelle, A désigne un atome d'hydrogène, ou un radical OR₂,
R₁ et R₂, identiques ou différents, désignent un radical acyle R₃CO dans lequel R₃ désigne un radical hydrocarboné en C₁-C₃₀, linéaire ou ramifié, saturé ou non saturé, hydroxylé ou non hydroxylé, carboxylé ou non carboxylé ; un radical PO (OX₁) (OX₂) ou un radical SO₂ (OX₃) dans lesquels, X₁, X₂, X₃, identiques ou différents, désignent un atome d'hydrogène ou un cation monovalent de métal alcalin ou d'NH4⁺ , X₁ et/ou X₂ pouvant également désigner un cation de métal divalent ; un radical uronyle ,
**R**_{**4**} et **R**_{**5**} identiques, désignent un atome d'hydrogène ou un radical hydroxyle.

Dans la formule (I) décrite ci-dessus, les radicaux alkyle peuvent être linéaires ou ramifiés et désigner notamment les radicaux méthyle, éthyle, propyle, isopropyle, n-propyle, butyle, n-butyle, tert-butyle ; préférentiellemnt le radical alkyle désigne le radical méthyle ; le radical glycosyle désigne un radical ose ou oside, tel que l'arabinose, le glucose, le fructose ou le saccharose. Préférentiellement le radical glycosyle désigne un radical glucosyle.
Dans la formule (I) décrite ci-dessus, le radical uronyle désigne plus particulièrement un radical mannuronyle ou glucuronyle.

Parmi les composés de formule (I) utilisables selon la présente invention, on peut citer en particulier le 3,5-dihydroxystilbène (ou Pinosylvine), ses éthers monométhylique et diméthylique, le 3,5,3',4'-tétrahydroxystilbène, et les formes mono- et di- glycosides correspondantes.
On peut également citer le 3-hydroxystilbène, et son éther méthylique.

Dans la présente invention, les formes glucosides et glucuronides de ces composés de formule (I) sont particulièrement intéressantes car, en raison de leur hydrosolubilité, elles présentent l'avantage d'être aisément formulables en milieu essentiellement aqueux. En outre, elles peuvent libérer in situ la substance active sous l'action des glycosidases d'origine bactérienne présentes au niveau des aisselles.

Selon la présente invention, on préfère utiliser plus particulièrement encore, le 3,5-dihydroxystilbène (ou Pinosylvine), qui, en comparaison du Triclosan bien connu dans le domaine considéré, présente l'avantage d'être plus efficace au niveau de l'activité déodorante, d'être inodore aux concentrations d'utilisation, et de mieux respecter la flore bactérienne cutanée, car son action bactéricide est sélective sur Corynebacterium xerosis, principale souche productrice des odeurs de transpiration.

Les composés de formule (I) selon l'invention représentent de préférence de 0,001 à 10% en poids environ du poids total de la composition cosmétique déodorante, plus particulièrement de 0,01 à 5%, et plus préférentiellement encore de 0,1 à 5% en poids environ de ce poids.

Les composés de formule (I) décrite ci-dessus dans laquelle A désigne un atome d'hydrogène, ou un radical OR₂,
R₁ et R₂, identiques ou différents, désignant un atome d'hydrogène ; un radical alkyle en C₁-C₄ ou un radical glycosyle, se trouvent à l'état naturel notamment dans les végétaux du genre :
   - Pinus et notamment Pinus montana mill., Pinus sylvestris L, Pinus contorta var. Latifolia S. Wats, Pinus ponderosa Dougl., Pinus Halpensis Mill. de la famille des Pinacées ;
   - Alnus, et notamment Alnus sieboldiana de la famille des Bétulacées ;
   - Polygonum, et notamment Polygonum nodosum, de la famille des Polygonacées ;
   - Dalbergia, et notamment Dalbergia sissoo, de la famille des Légumineuses ;
   - Scutellaria, et notamment Scutellaria scandens, de la famille des Lamiacées ;
   - Lindera, et notamment Lindera reflexa, de la famille des Lauracées.

Ainsi, le 3,5-dihydroxystilbène et ses éthers monométhylique et diméthylique peuvent être extraits des bois des végétaux du genre Pinus, le monoglucoside du 3,5-dihydroxystilbène peut être extrait des racines du genre Scutellaria ; le diglucoside du 3,5-dihydroxystilbène peut être extrait des végétaux du genre Lindera.
Le 3,5,3',4'-tétrahydroxystilbène (ou Piceatannol ou Astringenin) peut être extrait du bois des pins et épicéas et d'une légumineuse (Laburnum anagyroïdes).
Le 3,5-dihydroxystilbène, le 3-hydroxystilbène et le 3,5,3',4'-tétrahydroxystilbène peuvent provenir d'extraits végétaux, tels que décrits ci-dessus, et être obtenus par toute méthode d'extraction connue de l'homme du métier ou être obtenus par synthèse selon des méthodes généralement bien connues dans la littérature.
On peut en particulier citer les extraits alcooliques, notamment éthanoliques et les extraits hydroalcooliques.

La composition cosmétique déodorante selon l'invention peut comprendre, outre le ou les composés de formule (I) selon l'invention, d'autres actifs déodorants ou antitranspirants classiquement utilisés dans ce type de composition.

Au sens de la présente invention, on entend par actif déodorant, toute substance capable de réduire le flux sudoral et/ou masquer, améliorer ou réduire l'odeur désagréable résultant de la décomposition de la sueur humaine par les bactéries.

Ces actifs déodorants peuvent être choisis par exemple parmi :
des sels hydrosolubles de zinc, comme par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc ; des actifs antitranspirants tels que les sels d'aluminium, comme par exemple le chlorure d'aluminium et les hydroxyhalogénures d'aluminium, les sels de zirconium, comme par exemple des sels d'oxyde de zirconium ou des sels d'hydroxyzirconyle, les complexes de métaux comme l'aluminium ou le zirconium avec un acide aminé comme par exemple la glycine comme décrit dans US-3,792,068 ; des bactéricides tels que le 2,4,4'-trichloro-2'-hydroxydiphényléther (Triclosan), le 3,7,11-triméthyldodéca-2,5,10-triénol (Farnesol).
Ces actifs déodorants additionnels peuvent être présents dans la composition selon l'invention à raison d'environ 0,001 à 20% en poids par rapport à la composition totale, et de préférence à raison d'environ 0,1 à 10% en poids.

La composition cosmétique déodorante selon l'invention est formulée classiquement selon la forme de présentation à laquelle elle est destinée.
Elle est plus particulièrement formulée dans un véhicule cosmétiquement acceptable qui peut être notamment essentiellement aqueux, ou contenir des solvants organiques et notamment des monoalcools en C₁-C₄, de préférence de l'éthanol pour accélérer l'évaporation du produit, ou du propylène glycol, du dipropylène glycol ou leurs éthers.
La composition cosmétique déodorante selon l'invention peut également être formulée en émulsion eau-dans-huile, huile-dans-eau, ou en émulsion triple eau-dans-huile-dans-eau, (de telles émulsions sont connues et décrites par exemple par C. FOX dans 〈〈 Cosmetics and Toiletries 〉〉 - november 1986 - Vol 101 - pages 101-112).

La composition cosmétique déodorante de l'invention peut comprendre en outre des adjuvants cosmétiques choisis parmi les corps gras, les gélifiants, les émollients, les adoucissants, les antioxydants, les opacifiants, les stabilisants, les agents anti-mousse, les agents hydratants, les vitamines, les parfums, les conservateurs, les tensioactifs, les charges, les séquestrants, les polymères, les propulseurs, les agents alcalinisants ou acidifiants, les parfums, les colorants, les pigments, les agents épaississants, ou tout autre ingrédient habituellement utilisé en cosmétique pour ce type d'application.
Bien entendu, l'homme de métier veillera à choisir ce ou ces éventuels composes complémentaires de manière telle que les propriétés avantageuses attachées intrinséquement à la composition cosmétique déodorante conforme à l'invention ne soient pas, ou sustantiellement pas, altérées par la ou les adjonctions envisagees.

Les tensio-actifs sont choisis de préférence parmi les tensio-actifs anioniques, amphotères ou non-ioniques.
Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en C₁₂-C₁₅ (Finsolv TN de FINETEX), l'alcool myristique polyoxypropyléné à 3 moles d'oxyde de propylène (WITCONOL APM de WITCO), les triglycérides d'acides gras en C₆-C₁₈ tels que les triglycérides d'acide caprylique/caprique.
Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin (octanoate de stéaryle), les huiles de silicone et les isoparaffines.
Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MiO vendue par la société NL INDUSTRIES ou la Veegum Ultra, vendue par la société POLYPLASTIC.

La composition cosmétique déodorante peut comprendre des émollients, qui contribuent à une sensation douce, sèche, non-collante à l'application de la composition sur la peau. Ces émollients peuvent être choisis parmi des produits du type silicone volatile, des silicones non-volatiles et d'autres émollients non-volatils.
Les silicones volatiles sont définies de façon connue comme des composés volatils à température ambiante. On peut citer parmi ces composés les silicones volatiles cycliques et linéaires du type diméthylsiloxane dont les chaînes comprennent de 3 à 9 résidus siliconés. De préférence on choisit les cyclométhicones D4 ou D5.
Les silicones non-volatiles sont définies de façon connue comme des composés de pression de vapeur faible à température ambiante. Parmi ces composés sont inclus: les polyalkylsiloxanes, en particulier les polyalkylsiloxanes linéaires comme par exemple les polydiméthylsiloxanes, ou diméthicones, linéaires, commercialisés par la société Dow Corning sous le nom de 〈〈 Dow Corning 200 Fluid 〉〉 ; les polyalkylarylsiloxanes comme par exemple les polyméthylphénylsiloxanes, commercialisés par la société Dow Corning sous le nom de 〈〈 Dow Corning 556 Fluid 〉〉 ; les copolymères polyéther et siloxane, comme par exemple les diméthicone copolyols.
Parmi les émollients non-volatils utilisables dans la présente invention, on peut citer par exemple : les dérivés hydrocarbonés, les huiles minérales, les alcools gras, les esters d'alcools en C₃-C₁₈ avec des acides en C₃-C₁₈, les esters de l'acide benzoïque avec des alcools en C₁₂-C₁₈ et leurs mélanges, des polyols en C₂-C₆ choisis de préférence parmi le glycérol, le propylèneglycol ou le sorbitol, les polymères de polalkylène glycol.

Les quantités de ces différents constituants pouvant être présents dans la composition cosmétique déodorante selon l'invention sont celles classiquement utilisées pour les formes de présentation considérées.

La composition déodorante selon l'invention peut ainsi se présenter sous forme de lotion, de crème ou de gel fluide distribué en spray aérosol, en flacon pompe ou en roll-on, sous forme de crème épaisse distribuée en tube et sous forme de stick ou de poudre, et contenir à cet égard les ingrédients et propulseurs généralement utilisés dans ce type de produits et bien connus de l'homme de l'art.

Des exemples concrets, mais nullement limitatifs, illustrant l'invention vont maintenant être donnés.

### EXEMPLE 1 : Test in vitro d'évaluation d'odeurs

Comparaison de l'efficacité de la Pinosylvine (invention) contre celles d'actifs déodorants de l'art antérieur (Triclosan - Resvératrol - 4-hydroxystilbène).

On a réalisé un test d'inhibition de l'évolution d'odeurs sur de la sueur naturelle. Le principe de ce test consiste en l'ajout de l'agent déodorant à la sueur fraîche, puis à procéder à une évaluation olfactive par un jury d'experts après 18 heures et 24 heures d'incubation à 37°C.

On a donc prélevé en sauna les sueurs axillaires de plusieurs modèles humains que l'on a réunies pour réaliser un échantillon de sueur. Pour obtenir l'odeur nauséabonde caractéristique de la sueur, avant d'introduire la substance active, on a incubé cet échantillon de sueur pendant 18 heures à la température de 37°C. Dans chaque flacon de substance active à tester ainsi que dans un flacon témoin (sans substance active), on a introduit 1 ml de l'échantillon de sueur incubée. Puis on a introduit la substance active dans chacun des flacons à tester.
**1°)** Après 18 heures d'incubation, on a évalué l'odeur de chaque flacon.
L'odeur a été évaluée sur 2 critères :
(a)- l'intensité globale (notation de 0 à 4) :

| 0 | 1 | 2 | 3 | 4 |
|---|---|---|---|---|
| nulle | légère | modérée | forte | très forte |

(b)- l'appréciation hédonique (notation de 0 à 6) :

| 0 | 1 | 2 | 3 | 4 | 5 | 6 |
|---|---|---|---|---|---|---|
| très agréable | agréable | légèrement agréable | neutre | légèrement désagréable | désagréable | très désagréable |

**2°)** On a incubé à nouveau les flacons à l'étuve 37°C pendant 6h, le temps d'incubation total voulu étant de 24h.
On a répété ensuite l'évaluation d'odeur de chaque flacon comme précédemment.
**3°)** Résultats :
Les résultats ont été donnés en pourcentages de variation (réduction) de l'odeur et de la note hédonique par rapport à la sueur témoin sans agent déodorant :

| Agent déodorant | Quantité M.A. mg/ml de sueur | % moyen de réduction d'intensité d'odeur (T18h, T24h) | % moyen de réduction du désagrément d'odeur (T18h, T24h) |
|---|---|---|---|
| Pinosylvine | 1 | -77 | -33 |
| | 2 | -89 | -40 |
| Triclosan | 1 | -64 | -26 |
| | 2 | -64 | -29 |
| Resvératrol | 1 | -21 | -14 |
| | 2 | -25 | -12 |
| 4-hydroxystilbène | 1 | -18 | -3 |
| | 2 | -21 | -10 |

Les résultats démontrent que la Pinosylvine présente une réduction trés importante de l'intensité et du désagrément de l'odeur de la sueur naturelle pendant au moins 24h ; l'efficacité déodorante (réduction de l'intensité et du désagrément de l'odeur) de la Pinosylvine est supérieure à celle du Triclosan, du Resvératrol et du 4-hydroxystilbène; on remarquera que ces deux derniers composés ont une efficacité très insuffisante.

### EXEMPLE 2 : Détermination de l'activité biocide

La détermination de l'activité microbiostatique de la Pinosylvine a été effectuée comparativement au Triclosan et au Resvératrol (actifs déodorants de l'art antérieur), par la méthode des dilutions en milieu gélosé.

Chacun des 3 composés a été présenté sous forme de solution éthanolique.

Cent µl de la solution éthanolique ont été ajoutés à 1,9 ml de culture gélosé en surfusion à 45°C (milieu choisi en fonction des exigences nutritives de la souche étudiée). Après agitation Vortex, des dilutions successives, en progression géométrique de raison 2, de la solution obtenue ont été réalisées à l'aide du milieu de culture gélosé en surfusion à 45°C. Un ml de chaque dilution a été introduit dans les puits d'une microplaque (Flacon, 24 puits). Quatre µl de suspension microbienne ont été déposés à la surface du milieu gélosé.
Deux puits témoin ont également été préparés : H₂O 100 µl + 1,9 ml de milieu de culture (témoin de croissance), éthanol 100 µl + 1,9 ml de milieu de culture (témoin de non-toxicité de l'éthanol dilué au 1/20).

Après incubation dans des conditions (température, atmosphère et durée) fonction de la souche étudiée, la Concentration Minimale Inhibitrice (CMI) est donnée par la plus faible concentration de produit qui inhibe la croissance microbienne.

### Résultats : exprimés en µg/ml.

Pour les cupules témoin (H₂O ou éthanol), l'observation d'une croissance microbienne a permis d'exclure la présence d'un effet placebo.

| | CMI µg/ml | | |
|---|---|---|---|
| | Pinosylvine | Triclosan | Resvératrol |
| Staphylococcus aureus | 200 | 0,1 | 72 |
| Staphylococcus epidermis | 400 | 2,3 | 72 |
| Corynebacterium xerosis | 6 | 2,3 | 14 |
| Propionibacterium acnes | 200 | 2,3 | 29 |
| Pytirosporum ovale | >400 | >144 | >115 |
| Aspergillus niger | 400 | 18,1 | >115 |

Les résultats démontrent que :
(i) l'activité biocide de la Pinosylvine est inférieure à celle du Resvératrol, sur toutes les souches testées à l'exception de la souche Corynebacterium xerosis; l'activité bactéricide de la Pinosylvine s'exerce donc sélectivement.
(ii) l'activité biocide de la Pinosylvine est très nettement inférieure à celle du Triclosan sur toutes les souches testées.

### EXEMPLE 3 : Gel déodorant

| | |
|---|---|
| Behenoyl lactylate de sodium | 10,00 g |
| Glycérine | 73,10 g |
| Pinosylvine | 0,75 g |
| Ricinoléate de zinc | 1,00 g |
| Acide stéarique | 8,40 g |
| Hydroxyde de sodium | 1,20 g |
| Parfum, colorants | qs |
| Eau déminéralisée | qsp 100 g |

### EXEMPLE 4 : Emulsion eau-dans-silicone

| | |
|---|---|
| Silicone DC 245 Fluid (DOW CORNING) | 6,60 g |
| Silicone DC 5225 C (DOW CORNING) | 9,40 g |
| Alcool éthylique | 11,00 g |
| Propyléne glycol | 37,00 g |
| Pinosylvine | 0,50 g |
| Parfum, conservateurs, colorants | qs |
| Eau déminéralisée | qsp 100 g |

### EXEMPLE 5 : Stick aqueux

| | |
|---|---|
| Stéarate de sodium | 6,20 g |
| Pinosylvine | 1,00 g |
| Glycérine | 15,00 g |
| Propyléne glycol | 20,00 g |
| Parfum, conservateurs | qs |
| Eau déminéralisée | qsp 100 g |

### EXEMPLE 6 : Crème déodorante

| | |
|---|---|
| Mélange d'alcools cétylstéarylique et cétylstéarylique oxyéthyléné à 30 OE (Empiwax CL de la société ALBRIGHT & WILSON) | 7,00 g |
| Mélange de monostéarate et de distéarate de glycérol (Cerasynt SD-V de la société ISP) | 2,00 g |
| Huile de vaseline | 15,00 g |
| Glycérine | 20,00 g |
| Pinosylvine | 1,50 g |
| Parfum, conservateurs | qs |
| Eau déminéralisée | qsp 100 g |

### EXEMPLE 7 : Spray déodorant (flacon-pompe)

| | |
|---|---|
| Alcool éthylique 95% vol. dénaturé | 92,90 g |
| Ricinoléate de zinc | 2,00 g |
| Pinosylvine | 1,00 g |
| Parfum, colorant | qs |

### EXEMPLE 8 : Stick antitranspirant anhydre

| | |
|---|---|
| Alcool stéarylique | 22,00 g |
| Huile de ricin hydrogénée | 5,00 g |
| Palmitate d'isopropyle | 10,00 g |
| Chlorhydrate d'aluminium | 20,00 g |
| Pinosylvine | 3,00 g |
| Silicone D5 | 35,00 g |
| Talc | 5,00 g |

### EXEMPLE 9 : Spray déodorant (flacon pressurisé)

| | |
|---|---|
| Alcool éthylique 95% vol. dénaturé | 39,50 g |
| 3,5,3',4'-tétrahydroxystilbène | 0,50 g |
| Isobutane | 60,00 g |

## Revendications

1. Utilisation d'au moins un composé de formule (I) suivante : 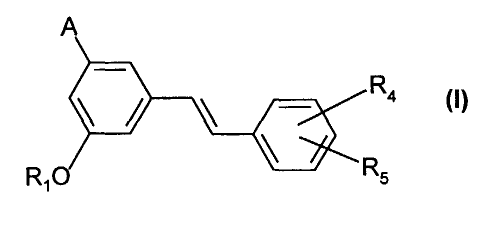 dans laquelle :
**A** désigne un atome d'hydrogène, ou un radical OR₂,
**R**_{**1**} et **R**_{**2**}, identiques ou différents, désignent un atome d'hydrogène ; un radical alkyle en C₁-C₄ ; un radical acyle R₃CO dans lequel R₃ désigne un radical hydrocarboné en C₁-C₃₀, linéaire ou ramifié, saturé ou non saturé, hydroxylé ou non hydroxylé, carboxylé ou non carboxylé ;
un radical PO (OX₁) (OX₂) ou un radical SO₂ (OX₃) dans lesquels, X₁, X₂, X₃, identiques ou différents, désignent un atome d'hydrogène ou un cation monovalent de métal alcalin ou d'NH4⁺ , X₁ et/ou X₂ pouvant également désigner un cation de métal divalent ;
un radical glycosyle ; un radical uronyle ;
**R**_{**4**} et **R**_{**5**} _{**,**} identiques, désignent un atome d'hydrogène ou un radical hydroxyle,
comme actif déodorant, dans une composition cosmétique.

2. Utilisation selon la revendication 1, caractérisée par le fait que dans la formule (I) le radical alkyle en C₁-C₄ désigne le radical méthyle.

3. Utilisation selon la revendication 1 ou 2, caractérisée par le fait que dans la formule (I) le radical glycosyle désigne le radical glucosyle.

4. Utilisation selon la revendication 1 ou 2, caractérisée par le fait que dans la formule (I) le radical uronyle est un radical mannuronyle ou glucuronyle.

5. Utilisation selon la revendication 1, caractérisée par le fait que le composé (I) est le 3,5-dihydroxystilbène (ou Pinosylvine).

6. Utilisation selon la revendication 1, caractérisée par le fait que le composé (I) est le 3-hydroxystilbène.

7. Utilisation selon la revendication 5, caractérisée par le fait que la Pinosylvine est un produit de synthèse.

8. Utilisation selon la revendication 5, caractérisée par le fait que la Pinosylvine est issue d'un extrait végétal la contenant.

9. Utilisation selon la revendication 8, caractérisée par le fait que l'extrait végétal est du genre :
- Pinus et notamment Pinus montana mill., Pinus sylvestris L, Pinus contorta var. Latifolia S. Wats, Pinus ponderosa Dougl., Pinus Halpensis Mill. de la famille des Pinacées ;
- Alnus, et notamment Alnus sieboldiana de la famille des Bétulacées ;
- Polygonum, et notamment Polygonum nodosum, de la famille des Polygonacées ;
- Dalbergia, et notamment Dalbergia sissoo, de la famille des Légumineuses ;
- Scutellaria, et notamment Scutellaria scandens, de la famille des Lamiacées ;
- Lindera, et notamment Lindera reflexa, de la famille des Lauracées.

10. Utilisation selon la revendication 1, caractérisée par le fait que le composé (I) est le 3,5,3',4'-tétrahydroxystilbène.

11. Composition cosmétique déodorante caractérisée par le fait qu'elle comprend, dans un véhicule cosmétiquement acceptable, au moins un composé de formule (I) tel que défini à l'une quelconque des revendications 1 à 10.

12. Composition selon la revendication 11, caractérisée par le ou les composés de formule (I), à titre d'actif déodorant, représentent de 0,001 à 10% de Matière Active en poids par rapport au poids total de la composition.

13. Composition selon la revendication 12, caractérisée par le fait que le ou les composés de formule (I) représentent de 0,01 à 5% de Matière Active en poids par rapport au poids total de la composition.

14. Composition selon la revendication 13, caractérisée par le fait que le ou les composés de formule (I) représentent de 0,1 à 5% de Matière Active en poids par rapport au poids total de la composition.

15. Composition selon l'une quelconque des revendications 11 à 14, caractérisée par le fait qu'elle comprend en outre au moins un actif déodorant ou antitranspirant additionnel différant du ou des composés de formule (I).

16. Composition selon l'une quelconque des revendications 11 à 15, caractérisée par le fait que le véhicule est essentiellement aqueux.

17. Composition selon la revendication 16, caractérisée par le fait que le véhicule contient en outre des solvants organiques.

18. Composition selon l'une quelconque des revendications 11 à 17, caractérisée par le fait qu'elle contient en outre des adjuvants cosmétiques choisis parmi les corps gras, les gélifiants, les émollients, les adoucissants, les antioxydants, les opacifiants, les stabilisants, les agents anti-mousse, les agents hydratants, les vitamines, les parfums, les conservateurs, les tensioactifs, les charges, les séquestrants, les polymères, les propulseurs, les agents alcalinisants ou acidifiants, les parfums, les colorants, les pigments, les agents épaississants, ou tout autre ingrédient habituellement utilisé en cosmétique.

19. Composition selon l'une quelconque des revendications 11 à 18, caractérisée par le fait qu'elle est formulée en émulsion eau-dans-huile, huile-dans-eau, ou en émulsion triple eau-dans-huile-dans-eau.

20. Composition selon l'une quelconque des revendications 11 à 19, caractérisée par le fait qu'elle se présente sous la forme de lotion, de crème ou de gel fluide distribué en spray aérosol, en flacon pompe ou en roll-on, sous forme de crème épaisse distribuée en tube et sous forme de stick ou de poudre.

21. Utilisation d'une composition selon l'une quelconque des revendications 11 à 20, comme, ou pour la fabrication de, produits cosmétiques déodorants destinés à l'application topique humaine.

22. Utilisation du 3,5-dihydroxystilbène sous forme synthétique ou d'extrait végétal le contenant comme agent inhibiteur sélectif de certaines espèces bactériennes constitutives de la flore cutanée humaine.

23. Utilisation du 3,5-dihydroxystilbène sous forme synthétique ou d'extrait végétal le contenant comme agent inhibiteur sélectif de l'espèce bactérienne Corynebacterium xerosis.

24. Procédé pour traiter les odeurs axillaires humaines, consistant à appliquer sur la surface axillaire une quantité efficace d'une composition selon l'une quelconque des revendications 11 à 20.

25. Composés de formule (I) telle que définie selon la revendication 1, et dans laquelle, A désigne un atome d'hydrogène, ou un radical OR₂, R, et R₂, identiques ou différents, désignent un radical acyle R₃CO dans lequel R₃ désigne un radical hydrocarboné en C₄-C₃₀, linéaire ou ramifié, saturé ou non saturé, hydroxylé ou non hydroxylé, carboxylé ou non carboxylé ; un radical PO (OX₁) (OX₂) ou un radical SO₂ (OX₃) dans lesquels, X₁, X₂, X₃, identiques ou différents, désignent un atome d'hydrogène ou un cation monovalent de métal alcalin ou d'NH4⁺ , X₁ et/ou X₂ pouvant également désigner un cation de métal divalent; un radical uronyle.

26. Composés selon la revendication 25, pour lesquels le radical uronyle est un radical mannuronyle ou glucuronyle.

27. Composition cosmétique comprenant au moins un composé de formule (I) telle que définie selon la revendication 1, et dans laquelle, A désigne un atome d'hydrogène, ou un radical OR₂,
**R**_{**1**} et **R**_{**2**}, identiques ou différents, désignent un atome d'hydrogène ; un radical alkyle en C₁-C₄ ; un radical acyle R₃CO dans lequel R₃ désigne un radical hydrocarboné en C₁-C₃₀, linéaire ou ramifié, saturé ou non saturé, hydroxylé ou non hydroxylé, carboxylé ou non carboxylé ;
un radical PO (OX₁) (OX₂) ou un radical SO₂ (OX₃) dans lesquels, X₁, X₂, X₃, identiques ou différents, désignent un atome d'hydrogène ou un cation monovalent de métal alcalin ou d'NH4⁺ , X₁ et/ou X₂ pouvant également désigner un cation de métal divalent ;
un radical glycosyle ; un radical uronyle ;
**R**_{**4**} et **R**_{**5,**} identiques, désignent un atome d'hydrogène ou un radical hydroxyle.

28. Composition cosmétique selon la revendication 27, caractérisé par le fait que le radical uronyle est un radical mannuronyle ou glucuronyle.
